(19) Europäisches Patentamt
European Patent Office
Office européen des brevets

(11) Veröffentlichungsnummer: **0 084 655**
**B1**

(12) # EUROPÄISCHE PATENTSCHRIFT

(45) Veröffentlichungstag der Patentschrift: **11.09.85**

(51) Int. Cl.⁴: **G 01 N 33/70**, G 01 N 33/53

(21) Anmeldenummer: **82111799.1**

(22) Anmeldetag: **20.12.82**

(54) **Verfahren und Reagenz zur Bestimmung von Creatinin.**

(30) Priorität: **22.12.81 DE 3150878**

(43) Veröffentlichungstag der Anmeldung: **03.08.83 Patentblatt 83/31**

(45) Bekanntmachung des Hinweises auf die Patenterteilung: **11.09.85 Patentblatt 85/37**

(84) Benannte Vertragsstaaten: **AT BE CH DE FR GB IT LI LU NL SE**

(56) Entgegenhaltungen:
**CLINICAL CHEMISTRY, Band 26, Nr. 8, 1980, Winston S.NARAYANAN et al. "Creatinine: A Review" Seiten 1119-1126**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Albert, Phil, Dr., Winfried, Moosstrasse 10, D-8121 Pähl (DE)**
Erfinder: **Ziegenhorn, Joachim, Dr. rer. nat., Ina-Seidel-Weg 1, D-8130 Starnberg (DE)**
Erfinder: **Siedel, Joachim, Dr. rer. nat., Bahnhofstrasse 6, D-8131 Bernried (DE)**
Erfinder: **Batz, Hans-Georg, Dr. rer. nat., Traubinger-Strasse 63, D-8132 Tutzing (DE)**
Erfinder: **Lenz, Helmut, Dr. rer. nat., Waldschmidtstrasse 7, D-8132 Tutzing (DE)**
Erfinder: **Pautz, Brigitte, Kientalstrasse 27, D-8036 Herrsching (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

**EP 0 084 655 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Bestimmung von Creatinin auf immunologischer Basis und ein hierfür geeignetes Reagenz. In der klinischen Chemie stellt die Bestimmung des Creatinins eine der wichtigsten Methoden zur Diagnostik der Nierenfunktion dar. Gegenüber der Harnstoffbestimmung hat sie den entscheidenden Vorteil, daß die Konzentration des Creatinins im Serum von der Ernährungsweise, insbesondere von der Zufuhr proteinreicher Nahrung, praktisch unbeeinflußt bleibt.

Allerdings ist im Vergleich zum Harnstoff die Konzentration des Creatinins im Serum im Entscheidungsbereich (Obergrenze der Normalwerte 1,10 mg/dl bei Männern bzw. 0,90 mg/dl bei Frauen) außerordentlich gering. Deshalb müssen an die Empfindlichkeit und Spezifität eines Creatinin-Tests hohe Anforderungen gestellt werden.

Wegen der Bedeutung des Creatinin-Tests als Standarduntersuchungsmethode im klinischen Labor sollte dieser aber gleichzeitig mit möglichst geringem Arbeitsaufwand durchführbar und besonders für den Einsatz an Analysenautomaten geeignet sein.

Die bisher gebräuchlichste Bestimmungsmethode für Creatinin beruht auf der von M. Jaffé gefundenen Farbreaktion von Creatinin mit Pikrinsäure im alkalischen Medium. Hierbei wird nach saurer Enteiweißung der Probe (z. B. mit Trichloressigsäure oder mit Pikrinsäure) im Überstand nach Zugabe von Pikrinsäure und Alkalisieren eine rote Färbung entwickelt und photometrisch gemessen. Dieses an sich einfache Verfahren ist jedoch mit einer Reihe wesentlicher Nachteile behaftet.

Es hat sich gezeigt, daß die Jaffé-Reaktion von über 50 ebenfalls chromogenen Substanzen beeinflußt wird [Literatur: Clin. Chem. 26, 1119—1126 (1980)], insbesondere von natürlicherweise im Serum vorkommenden Bestandteilen wie Glucose, Pyruvat, Acetoacetat und Aceton und somit nicht für Creatinin spezifisch ist. Diese »Nichtcreatinin Chromogene« stören vor allem bei niedrigen Creatininkonzentrationen ($\leq$1 mg/dl) was zu einer Limitierung der unteren Erfassungsgrenze für Creatinin führt (»creatininblinder« Bereich).

Auch führen schon geringe Verschiebungen des pH-Wertes im Reaktionsmilieu zu einer Veränderung der Farbtiefe. Schließlich stellt auch die Verwendung teilweise ätzender und giftiger Reagenzien eine Gefahrenquelle bei der Handhabung dar. Eine Reihe von Modifikationen der Jaffé-Reaktion verbessert zwar die Präzision und Durchführbarkeit, ohne jedoch diese prinzipiellen Mängel vollständig zu beheben.

Eine weitere, bekannte Methode wandelt Creatinin unter Zusatz von o-Nitrobenzaldehyd in Methylguanidin um, welches dann nach der Sakaguchi-Reaktion bestimmt wird. Bekannt ist auch eine Farbreaktion zwischen Creatinin und Kalium-Quecksilber-Thiocyanat. Beide Methoden erwiesen sich jedoch für das klinische Labor als ungeeignet.

Bekannt ist ferner, durch Kombination der Jaffé-Reaktion mit enzymatischen Teilschritten Störungen durch unspezifische Chromogene zu umgehen. Dabei wird die mit der Serumprobe vor und nach Behandlung mit Creatinin-amidohydrolase/Creatininkinase/ATP erhaltene Färbung in der Jaffé-Reaktion bestimmt und aus der Differenz der Extinktionen der Creatinin-Gehalt berechnet (Arch. Pharm. 3, 893—896 (1980)).

Diese Methode ist zwar spezifisch, die Durchführung jedoch sehr umständlich und nur schwer automatisierbar.

Ferner sind Verfahren zur Creatininbestimmung bekannt, in denen der aus Creatinin durch Einwirkung der Creatinin-Iminohydrolase freigesetzte Ammoniak bestimmt wird, entweder mit ammoniakselektiven Elektroden (Anal. Chem. 46, 246—249 (1976)) oder fluorimetrisch über den NADH-Verbrauch in der nachgeschalteten Glutamatdehydrogenase-Reaktion (Clin. Chim. Acta 100, 21—23, (1980)). Wegen der möglichen Gegenwart vergleichsweise großer Mengen an freiem Ammoniak im Probenmaterial erscheint es jedoch fraglich, ob derartige Messungen hinreichend störungsfrei gestaltet werden und damit Eingang in die Routinediagnostik finden können.

In neuerer Zeit wurde ein vollenzymatischer Creatinintest beschrieben, bei dem Creatinin in Creatin umgewandelt, letzteres mit ATP zu Creatinphosphat umgesetzt und das dabei entstehende ADP in einer gekoppelten Reaktion mit Pyruvatkinase und Lactatdehydrogenase (LDH) photometrisch über die Abnahme des NADH-Gehaltes in der Reaktionslösung gemessen wird [Scand. J. clin. Lab. Invest., suppl. 29, p. 126 (1972)].

Diese Methode erfordert keine Enteiweißung der Serumprobe und ist spezifisch für Creatinin. Wegen des relativ geringen Meßsignals, selbst bei Einsatz größerer Probevolumina, ist jedoch die Empfindlichkeit des Tests im niedrigen Creatinin-Konzentrationsbereich limitiert; zudem ist durch die Notwendigkeit einer Probenleerwertbestimmung eine Anwendung der Methode in Analysenautomaten sehr erschwert.

Es besteht daher der Bedarf an einem einfachen und automatisierbaren, gleichzeitig sehr spezifischen und aus den obengenannten Gründen ganz besonders im Konzentrationsbereich $\leq$1 mg/dl Creatinin (»creatininblinder Bereich«) sehr empfindlichen Test für Creatinin.

Die Erfindung löst dieses Problem durch ein immunologisches Verfahren zur Bestimmung von Creatinin, welches darauf beruht, daß das Creatinin aus der Probe zunächst in Methylhydantoin, vorzugsweise enzymatisch mit Hilfe der Creatinin-Iminohydrolase (EC. 3.5.4.21), umgewandelt wird und durch das entstandene 1-Methylhydantoin die Bindungsreaktion zwischen einem Konjugat eines

Hydantoins der allgemeinen Formel I

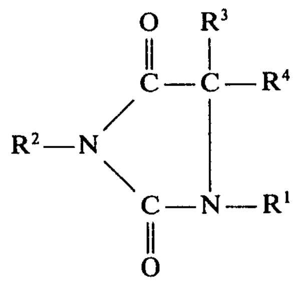

in der $R^1$, $R^2$, $R^3$ und $R^4$ unabhängig voneinander je ein H-Atom, einen Alkylrest mit 1 bis 3 C-Atomen oder einen Phenylrest bedeuten, mit einer Haptenträgersubstanz und gegen ein Konjugat eines weiteren Hydantoins der allgemeinen Formel I mit derselben, bevorzugt aber mit einer anderen Haptenträgersubstanz gerichteten Antikörpern, z. B. in Form von Antiserum bzw. der daraus gewonnenen Immunglobulinfraktion, in konzentrationsabhängiger Weise gehemmt wird.

In einer bevorzugten Ausführungsform werden sowohl für den Bindungshemmtest als auch für die Antikörpergewinnung Konjugate von Haptenträgersubstanzen mit 1-Methylhydantoin ($R^1 = CH_3$, $R^{2-4} = -H$) eingesetzt.

Die Bindungshemmung zwischen Antikörper und Hydantoin-Konjugat ist umso stärker, je mehr Creatinin und damit 1-Methylhydantoin dem Antikörper vor dem Vermischen mit dem Hydantoin-Konjugat zugesetzt wurde. Der Hemmungseffekt ist bereits in einem Konzentrationsbereich des Creatinins in der zu messenden Probelösung unter 1 mg/dl, also dem Bereich, in dem die Brauchbarkeit der bekannten Creatinin-Bestimmungsmethoden bereits sehr eingeschränkt ist, stark ausgeprägt.

Obwohl seit langer Zeit ein Bedarf an einer einfachen und spezifischen, insbesondere im niedrigen Konzentrationsbereich verbesserten Creatinin-Bestimmungsmethode bestanden hat und auch immunologische Testverfahren seit 40 Jahren bekannt sind, ist es bisher nicht gelungen, eine immunologische Bestimmungsmethode für Creatinin aufzufinden.

Dies dürfte unter anderem darauf zurückzuführen sein, daß Creatinin eine im Körper aller Organismen, die Antikörper bilden, weit verbreitete Substanz darstellt, die selber eine Antikörperbildung nicht auslösen kann und auch wegen der geringen Molekülgröße und der verhältnismäßig hohen Serumkonzentration nicht zu erwarten war, daß selbst ein Konjugat von Creatinin mit einer Haptenträgersubstanz die Bildung von Antikörpern verursachen kann, die nicht einmal mit dem Creatinin strukturell eng verwandten Verbindungen, wie z. B. Creatin, kreuzreagieren, und sich damit eine spezifische Bestimmungsmethode für Creatinin aufbauen ließe.

Die Erfindung umgeht diese Probleme dadurch, daß aus dem Creatinin zunächst in einer »Verfremdungsreaktion« ein Hydantoin gebildet wird, welches seinerseits eine zwar auch niedermolekulare, aber auch körperfremde Verbindung darstellt, gegen die mit geeigneten Hydantoin-Konjugaten, insbesondere mit 1-Methylhydantoin-Haptenträgersubstanz-Konjugaten, Antikörper erzeugt werden können, die überraschenderweise so spezifisch sind, daß sie nicht einmal in signifikantem Ausmaß mit körpereigenen Verbindungen kreuzreagieren, bei denen wesentliche Strukturmerkmale mit denen des Hydantoins, wie z. B. Creatinin, Creatin und Harnstoff, gemeinsam sind, und die sich deshalb unter Zuhilfenahme der obengenannten Verfremdungsreaktion zum Aufbau eines spezifischen, empfindlichen Creatinin-Tests eignen.

Als zur Antikörperbildung geeignete Haptenträgersubstanzen eignen sich die in der Immunologie hierfür bekannten Stoffe. Beispiele hierfür sind im Prinzip alle artfremden Proteine, die im Wirtstier, welches zur Antikörperbildung herangezogen wird, nicht vorkommen, wie Serumalbumine verschiedenen Ursprungs, Key-hole-lymphocyanin, (Lipo)-Polysaccharide, Agarose, Aktivkohle, Viren. Beispiele für weitere bekannte Trägersubstanzen für Haptene sind in Handbook of Experimental Immunology, Blackwell Scientific Publications, 3. Aufl. (1978), S. 1—11, angegeben. Dort finden sich auch geeignete Methoden zur Verknüpfung von Trägersubstanz und Hapten. Bevorzugte Haptenträgersubstanzen im Rahmen der Erfindung sind Serumalbumine verschiedener Herkunft wie z. B. Rinder- oder Humanserumalbumin und dergleichen, sowie Edestin.

Enthält das zur Antikörperbildung verwendete Konjugat dieselbe Haptenträgersubstanz wie das im Bindungshemmtest verwendete Konjugat, so können in diesem Fall Kreuzreaktionen gegen Haptenträgersubstanz im Test dadurch selektiv kaschiert werden, daß man der Antikörperfraktion vor Bestimmung der eigentlich interessierenden Bindungsreaktion mit dem Hapten die Haptenträgersubstanz in der zur Fällung der gegen die Haptenträgersubstanz gerichteten Antikörper notwendigen Konzentration zusetzt, wobei bei der Durchführung des Tests über die Bestimmung der Trübungsbildung (TINIA, NINIA) die zunächst durch die Kreuzreaktion der Hydantoin-Konjugat-Antikörper mit der Haptenträgersubstanz entstandenen Niederschläge entfernt werden, z. B. durch Zentrifugation oder Filtration.

Die zur Immunisierung bzw. im Bindungshemmtest verwendeten Hydantoin-Konjugate werden bevorzugt dadurch hergestellt, daß man das Hydantoin an eine aliphatische oder araliphatische Carbonsäure bindet und dann die Carboxylgruppe des von der Carbonsäure stammenden Teils mit einer

Haptenträgersubstanz verknüpft. Als geeignet erwiesen sich hierfür die Fettsäuren mit mindestens 2 Kohlenstoffatomen, vorzugsweise mit 4 bis 16 Kohlenstoffatomen sowie Alkylseitengruppen tragende aromatische Carbonsäuren. Besonders geeignet sind Benzoesäurederivate mit einer Alkylgruppe von 1 bis 4 Kohlenstoffatomen am aromatischen Ring. Typische Beispiele sind Methylbenzoesäure, Äthylbenzoesäure und Propylbenzoesäure. Ebenfalls geeignet sind die Oligomeren dieser araliphatischen Carbonsäuren wie z. B. Methylbenzoat-methylbenzoesäure.

Die Bindung zwischen dem Hydantoin, das hier immunologisch als Hapten anzusehen ist, und der Carbonsäure kann nach an sich bekannten Methoden unter Verwendung aktivierter Carbonsäurederivate hergestellt werden. Bevorzugt verwendet man halogenierte Carbonsäuren, insbesondere die $\omega$-Bromcarbonsäuren in wäßrig alkoholischem Medium, oder im Falle der entsprechenden halogenierten Carbonsäureester in wasserfreiem Medium mit anschließender Verseifung. Die besten Ergebnisse wurden mit den $\omega$-Bromcarbonsäureestern mit anschließender Verseifung erzielt, wobei Ausbeuten bis 50% erhalten wurden. In wäßrig alkoholischem Medium erfolgt die Umsetzung mit dem Hydantoin bei erhöhter Temperatur, zweckmäßig am Siedepunkt. Verwendet man die Carbonsäureester, so werden als Lösungsmittel zweckmäßig polare organische Verbindungen wie Dimethylformamid, Formamid, Tetrahydrofuran und dergleichen verwendet. Die Umsetzung erfolgt zweckmäßig aus dem Natirumsalz des Hydantoins, welches durch Umsetzung mit Methanolat gewonnen wird. Die Verseifung des Produktes kann unter gelindem Erwärmen in wäßrig alkalischem Medium durchgeführt werden. Die Kupplung kann außer über den (unsubstituierten) Stickstoff in 3-Stellung des Hydantoins auch über den Kohlenstoff in 5-Position ($R^3$ und $R^4 = -H$) durch Diazotierung mit p-Benzoesäure-diazoniumsalz erfolgen. Bevorzugt werden zur Immunisierung sowie im Bindungshemmtest über den Stickstoff in 3-Stellung verbrückte Hydantoin-Konjugate.

Die Kupplung mit der Haptenträgersubstanz erfolgt zweckmäßig in wäßrig organischem Medium in Gegenwart eines Amins und eines Chlorameisensäureesters oder durch Herstellung des Hydroxysuccinimidesters der Carbonsäuren und anschließender Reaktion dieses Esters mit Proteinen oder anderen Trägern bei pH = 7 bis 9. Als besonders geeignet erwies sich Wasser/Dioxan als Reaktionsmedium.

Die so erhaltenen Hydantoin-Konjugate werden als Immunogen zur Antiserumbildung bzw. direkt im Bindungshemmtest verwendet.

Zur Antiserumbildung werden sie nach dem Fachmann bekannten Methoden der ausgewählten Tierspezies appliziert. Vorzugsweise wird das Immunogen zusammen mit dem Freund'schen Adjuvanz zur Verstärkung der Immunantwort verwendet.

Für die Antikörperbildung können generell alle antikörperbildenden Lebewesen verwendet werden. Bevorzugt werden Schafe verwendet. Auch monoclonale Antikörper, wie sie aus Zellkulturen erhältlich sind, kommen in Betracht.

Unter dem Begriff Antikörper werden im Rahmen der vorliegenden Erfindung sowohl gereinigte Antikörper als auch Antiseren, aus letzteren gewonnene Immunglobulinfraktionen sowie Antikörperfragmente, wie $F(ab')_2$, Fab- und Fv-Fragmente verstanden.

Die Hemmungswirkung des 1-Methylhydantoins auf die Bindungsreaktion zwischen Hydantoin-Konjugat und Hydantoin-Konjugat-Antikörpern kann nach an sich bekannten immunologischen Methoden direkt gemessen werden. Als Beispiele seien erwähnt die Methoden unter Verwendung markierter Antikörper bzw. Antigene, wie RIA, EIA, letzteres mit den Ausführungsformen ELISA oder EMIT®, die turbidimetrische Messung der Hemmung der Immunpräzipitation zwischen Konjugat und Antikörper (TINIA-Prinzip) bzw. die entsprechende nephelometrische Methode (NINIA-Prinzip) und ähnliche.

Ferner sind anwendbar Agglutinations-Inhibitions-Tests (PACIA® = Particel Counting Immuno-Assay) und Komplementbindungsreaktionen. Diese Methoden sind dem Fachmann sämtlich bekannt und brauchen hier nicht näher beschrieben zu werden. Lediglich beispielsweise wird für Ausführungsformen des EIA auf Clin. Chim. Acta, 81 (1977), S. 1—36 und J. Clin. Chem. Clin. Biochem. 18 (1980), S. 197—208 verwiesen.

Im Falle der Enzymmarkierung können die hierfür erfolgreich verwendeten Enzyme, wie $\beta$-Galactosidase, Peroxidase, alkalische Phosphatase, Glucoseoxidase, Glucose-6-phosphat-dehydrogenase und Luciferase erwähnt werden. Für eine Markierung mit Coenzym kommen z. B. NAD, NADP bzw. deren reduzierte Stufen in Betracht. Markiert kann sowohl Antikörper als auch Hapten sein.

Eine bevorzugte Methode unter den oben genannten Methoden besteht darin, daß man die Hemmung der Bindungsreaktion durch turbidimetrische Messung der Immunpräzipitation in einem vorgegebenen Zeitintervall bestimmt.

Eine weitere bevorzugte Methode besteht darin, daß man die Hemmung der Bindungsreaktion durch Rücktitration von nichtgebundenem Haptenträgersubstanz-Hydantoin-Konjugat mit markierten Antikörpern, besonders bevorzugt enzymmarkierten Antikörpern, und Messung des gebundenen oder des nichtgebundenen Anteils der markierten Substanz bestimmt. Hierfür eignet sich z. B. die Markierung mit radioaktiven Substanzen (RIA), Enzymen bzw. Coenzymen (EIA), Fluoreszenz (FIA), Spinmarkierung (vgl. Nature New Biology, Vol. 236 (1972), S. 93/94. Als markierte Antikörper können auch markierte Anti-Antikörper verwendet werden (Doppelantikörpermethode).

Ein weiterer Gegenstand der Erfindung ist ein Reagenz zur immunologischen Bestimmung von Creatinin, welches dadurch gekennzeichnet ist, daß es Creatinin-Iminohydrolase, Antikörper gegen

ein Konjugat eines Hydantoins, vorzugsweise des 1-Methylhydantoins, mit einer ersten Haptenträgersubstanz, ein Konjugat eines Hydantoins, vorzugsweise des 1-Methylhydantoins mit einer zweiten Haptenträgersubstanz, die mit der ersten Haptenträgersubstanz nicht wesentlich kreuzreagiert, und Puffersubstanz enthält.

Das erfindungsgemäße Reagenz kann auch eine Substanz enthalten, welche die Immunpräzipitation fördert. Besonders geeignet ist hierfür Polyäthylenglykol, allein oder gegebenenfalls zusammen mit einer oberflächenaktiven Substanz. Als Polyäthylenglykol eignet sich ein solches mit einem Molekulargewicht zwischen 200 und 20 000, bevorzugt wird ein Molekulargewicht von 6000 ± 2000. Geeignete Konzentrationen für das Polyäthylenglykol im Reagenz liegen zwischen 0 und 8%, vorzugsweise zwischen 1 und 4%.

Hinsichtlich des Antikörperbestandteils und der angewandten Markierung gelten die obigen Ausführungen für das erfindungsgemäße Reagenz entsprechend.

Für die turbidimetrische Bestimmungsmethode wird erfindungsgemäß ein Reagenz bevorzugt, welches 0,05 bis 100 U/ml Creatinin-Iminohydrolase, 0,5 bis 500 µg/ml Konjugat aus 1-Methylhydantoin und Human- oder Rinderserumalbumin, verbrückt über eine araliphatische Carbonsäure wie p-Methylbenzoesäure, im molaren Verhältnis 1-Methylhydantoin : Serumalbumin von 2 : 1 bis 30 : 1, Antikörper gegen ein Konjugat von 1-Methylhydantoin mit Edestin, verbrückt über eine aliphatische Carbonsäure wie Buttersäure, im Molverhältnis 0,1 bis 10, bezogen auf Hapten-Konjugat-Bindungsstellen, 0 bis 8% Polyäthylenglykol, sowie 0,05 bis 1 Mol/1 Puffersubstanz pH 4 bis 10, enthält.

Für die EMIT®-Methode enthält das erfindungsgemäße Reagenz vorzugsweise $10^{-4}$ bis $10^{-14}$ Mol/l Antikörper gegen 1-Methylhydantoin-Haptenträgersubstanz-Konjugat, bezogen auf aktive Rezeptorstellen, $10^{-4}$ bis $10^{-14}$ Mol/l 1-Methylhydantoin-Malatdehydrogenase-Konjugat, 0,05 bis 100 U/ml Creatinin- Iminohydrolase, 0,05 bis 50 mMol/l Oxalessigsäure, 5 bis 200 mMol/l Phosphatpuffer, pH 6 bis 8,5, und 0,05 bis 0,4 mMol/l NADH.

Als Puffersubstanzen können im Rahmen der Erfindung die bekannten, im pH-Bereich von 4 bis 10, vorzugsweise 6 bis 9, wirksamen verwendet werden. Die Pufferkonzentration im gelösten Reagenz sollte zwischen 0,005 und 1,0 Mol/l, vorzugsweise zwischen 0,01 und 0,2 Mol/l liegen. Besonders bevorzugt wird der Bereich von 0,03 bis 0,07 Mol/l.

Die Antikörperkonzentration im Test sollte zweckmäßig zwischen etwa $10^{-4}$ und $10^{-14}$ Mol/l, vorzugsweise zwischen $10^{-6}$ und $10^{-12}$ Mol/l liegen, wobei hier unter Mol/l aktive Rezeptorstellen verstanden werden.

Zur Durchführung des Verfahrens der Erfindung kann die zu analysierende Lösung unmittelbar dem Reagenz zugesetzt werden. Liegt das Creatinin in hohen Konzentrationen vor, wird die Probe vorher zweckmäßig mit Wasser verdünnt.

Die Bestimmung kann im allgemeinen bei Temperaturen zwischen 10 und 50°C, insbesondere zwischen 15 und 40°C durchgeführt werden.

Die Herstellung der Hydantoin-Konjugate kann nach bekannten Methoden erfolgen, beispielsweise nach der in J. Biol. Chem. 228, (1957) S. 713—727, beschriebenen Arbeitsweise der gemischten Anhydride. Gleiches gilt für die Markierung mit einem Enzym.

Die folgenden Beispiele erläutern die Erfindung weiter. Die darin verwendeten Abkürzungen haben die nachstehend angegebene Bedeutung:

| | |
|---|---|
| RSA | Rinderserumalbumin |
| HSA | Humanserumalbumin |
| RIA | Radioimmunoassay |
| EMIT® | Enzyme multiplied immunoassay technique |
| ELISA | Enzyme linked immunosorbent assay |
| TINIA | Turbidity inhibition immunoassay |
| NINIA | Nephelometric inhibition immunoassay |
| BP | Beschichtungspuffer |
| IP | Inkubationspuffer |
| Tween®20 | Polyoxyethylen-sorbitanmonolaurat |
| DMF | Dimethylformamid |
| WP | Waschpuffer |
| SP | Substratpuffer |
| PNPP-Na | p-Nitrophenylphosphat-Na |
| RT | Raumtemperatur |
| AP | Alkalische Phosphatase |
| AP-TC | Alkalische Phosphatase-Testcombination |

Tinaquant®-FN-Puffer:

| | | |
|---|---|---|
| | Na, K-Phosphat | 66 mMol/l pH 8,0 |
| | EDTA | 10 mMol/l |
| | Brij®-35 | 0,4% |
| | $NaN_3$ | 0,1% |
| | Polyethylen-glykol 6000 | 3,0% |
| EIA | Enzym Immunoassay | |

A) Herstellung der Hydantoin-Konjugate

11,4 g (100 mMol) 1-Methylhydantoin werden in heißem absoluten Methanol gelöst und mit der äquivalenten Menge Natrium-Methanolat versetzt. Das Methanol wird danach abdestilliert, der zurückbleibende Rückstand im Vakuum getrocknet und anschließend in 200 ml DMF aufgenommen. Es wird auf 80 bis 100°C erwärmt. Zu dieser Lösung werden unter Rühren langsam entweder 120 mMol/l $\gamma$-Brombuttersäureethylester oder p-Brommethylbenzoesäure hinzugefügt. Danach wird noch weitere 5 Stunden bei 100°C gerührt, dann abgekühlt und vom entstandenen Natriumbromid abfiltriert. Das Filtrat wird eingeengt und in 100 ml Isopropanol aufgenommen. Danach wird von erneut ausgefallenem Natriumbromid abfiltriert. Das Filtrat wird auf ein Viertel eingeengt und auf 4°C abgekühlt, wobei die weiße Substanz auskristallisiert. Das Kristallisat wird aus Wasser/Methanol (1 : 1) umkristallisiert. Der entstandene Ester wird mit 1 N NaOH verseift. Die freie Säure wird aus Isopropanol umkristallisiert.

1. 1-Methyl-3-(3-carboxypropyl)-hydantoin: Fp 102 bis 104°C, Ausbeute 61%
2. 1-Methyl-3-(p-carboxyphenyl)-methyl-hydantoin: Fp 167 bis 170°C, Ausbeute 67%.

Zur Kupplung an Rinderserumalbumin werden 3,6 g Rinderserumalbumin in 250 ml Wasser/Dioxan (1 : 1) angelöst, 5,6 ml I N NaOH zugegeben, wodurch Lösung eintritt, und anschließend auf 4°C gekühlt. Zu dieser Lösung wird eine Lösung von 1,7 g l-Methyl-3-(p-carboxyphenyl)-methyl-hydantoin, 1,7 ml Tributylamin und 0,95 ml Chlorameisensäureisobutylester in 60 ml Dioxan und 3 ml DMF zugetropft. Der Ansatz wird 24 Stunden bei 4°C gerührt, dann gegen fließendes, entsalztes Wasser 36 Stunden lang dialysiert. Danach wird die Lösung lyophilisiert.

Zur Kupplung an Edestin wird wie oben verfahren, nur daß statt Rinderserumalbumin Edestin und statt 1-Methyl-3-(p-carboxyphenyl)-methyl-hydantoin 1-Methyl-3-(3-carboxypropyl)-hydantoin eingesetzt werden.

B) Herstellung des Antiserums

Die Immunisierung der für die Antiserumgewinnung benutzten Tiere wird wie folgt durchgeführt:

Tierspecies: Schaf

Immunogen:
1-Methyl-3-3-(3-carboxypropyl)-hydantoin-Edestin-Konjugat

Immunisierungsschema:

| Tag | Applikation | Immunogenmenge | Freund-Adjuvans emulgiert mit |
|---|---|---|---|
| 0 | intradermal | 1 mg | + |
| 7 | intramuskulär | 1 mg | + |
| 14 | subcutan | 1 mg | + |
| 30 | intramuskulär | 1 mg | + |
| 60 | subcutan | 1 mg | + |
| usw. | subcutan | 1 mg | + |

1. Probeblutung: Tag 45

C) Aufbereitung des Antiserums

Schaf-Antiserum wird bei RT mit 1% Aerosil® (amorphe Kieselsäure) versetzt, ca. 2 Stunden gerührt, abzentrifugiert und der Niederschlag verworfen.

Zum Überstand wird langsam festes Ammoniumsulfat bis auf 1,8 Mol/l zugefügt und mehrere Stunden bei 4° C gerührt. Die Mischung wird abzentrifugiert und der Überstand verworfen. Der Niederschlag wird in 75% des Ausgangsvolumens aufgenommen (50 mMol/l K-Phosphat, pH 7,0, 100 mMol/l NaCl, 0,05% $NaN_3$), dann ca. 24 Stunden gegen 0,15 Mol/l NaCl dialysiert und anschließend gegebenenfalls abzentrifugiert.

Der Überstand wird dann ca. 8 bis 10 Stunden gegen 3 mMol/l HCl dialysiert, mit einem Wechsel der Dialyse-Lösung (1 : 100 Volumenverhältnis). Entstandene Niederschläge werden abzentrifugiert und verworfen.

Anschließend erfolgt eine Rückdialyse gegen 10 mMol/l K-Phosphat, pH 7,0, 150 mMol/l NaCl über ca. 12 Stunden (Volumenverhältnis 1 : 100). Entstandene Niederschläge werden abzentrifugiert und verworfen.

Beispiel 1

TINIA-Prinzip

a) Reagenzien:

Creatinin-Lösungen: Konzentrationen von 0,2 bis 1000 mg/dl Tinaquant®-FN-Puffer
Creatinin-Iminohydrolase
Antiserum gegen 1-Methyl-3-(3-carboxypropyl)-hydantoin-Edestin-Konjugat, 1 : 5 mit Tinaquant®-FN-Puffer verdünnt, von Trübungen durch Zentrifugation befreit.
1-Methyl-3-(p-carboxyphenyl)-methyl-hydantoin-RSA-Konjugat
(2 mg/ml Tinaquant®-FN-Puffer)

b) Testansatz:

In Küvetten (d = 1 cm) werden zu je 1 ml verdünntem Antiserum, enthaltend 10 U Creatinin-Iminohydrolase, 10 µl der Creatinin-Lösung verschiedener Konzentrationen bzw. 10 µl Tinaquant®-FN-Puffer für den Leerwert hinzupipettiert und 10 min bei 25° C inkubiert.

Anschließend werden jeweils 10 µl der Methylhydantoin-RSA-Konjugatlösung hinzupipettiert und die Zunahme der Trübung wird photometrisch bei 366 nm nach Zugabe des Methylhydantoin-RSA-Konjugats gemessen ($E_1$ vor Start, $E_2$ 5 Minuten nach Start). Figur 1 der Zeichnung zeigt eine so ermittelte Eichkurve, in der die Extinktionsdifferenz gegen die Creatininkonzentration aufgetragen ist.

Beispiel 2

ELISA-Prinzip
(in Anlehnung an das Verfahren aus J. of Immunology, 123, (1979), 1548—1550)

a) Reagenzien:

Beschichtungspuffer (BP)
0,2 Mol $Na_2CO_3$/l, pH 9,3 bis 9,5

Inkubationspuffer (IP)
0,05 mol/l K-Phosphat pH 7,2
0,1 Mol/l NaCl
1% Glycerin
0,05% Tween®20
0,02% $NaN_3$

Waschpuffer (WP)
0,15 Mol/l NaCl
0,05% Tween®20
0,02% $NaN_3$

Substratpuffer (SP)
AP-Test
1 Tablette/76 ml Puffer oder
17 mg PNPP-$Na_2$/10 ml Puffer aus AP-Testkombination
(Boehringer Mannheim GmbH, Best. Nr. 123 854)

Konjugat aus AP mit Kaninchen-Anti-Schaf-IgG

Creatinin-Iminohydrolase

b) Testansatz:

Mikrotiterplatten werden mit 200 µl Methylhydantoin-RSA-Konjugat (0,50 µg/ml BP bzw. 0,50 µg RSA/ml BP für den Leerwert) beschichtet, bei RT ca. 16 Stunden inkubiert und dann ausgesaugt. Dann werden 300 µl IP zugegeben, inkubiert (1 Stunde) nochmals ausgesaugt, danach 300 µl Waschpuffer zugegeben und erneut ausgesaugt. Anschließend erfolgt die Beladung mit Methylhydantoin-Antiserum. Hierzu werden 1000 µl Antiserum (1 : 200 bis 1 : 2000 in IP verdünnt), enthaltend 10 U/ml Creatinin-Iminohydrolase, mit 10 µl Creatinin-Probe bzw. 10 µl IP (für den Leerwert) 30 Minuten bei RT inkubiert, dann 200 µl der so erhaltenen Antiserumverdünnung auf die beschichteten Mikrotiterplatten gegeben, 60 Minuten verschlossen (Plastikbeutel) stehengelassen, ausgesaugt, 300 µl WP zugegeben und erneut ausgesaugt.

Zur Konjugatbeschichtung werden nunmehr 200 µl Kaninchen-Anti-Schaf-IgG-AP-Konjugat enthaltend 150 mU AP/ml IP auf die behandelten Mikrotiterplatten gegeben, für 2 Stunden bei 37° C gehalten, dann ausgesaugt und mit je 300 µl WP zweimal gewaschen.

Zur Farbentwicklung werden 200 µl Substratpuffer zugesetzt und die Mischung 30 bis 60 Minuten inkubiert. Die Auswertung erfolgt, indem man 150 µl Testlösung aus den Mikrotiterplatten mit 500 µl NaOH, 0,1 Mol/l, bei 405 nm gegen Leerwert mißt. Fig. 2 der Zeichnung zeigt eine mit verschiedenen Creatinin-Konzentrationen erhaltene Eichkurve.

Beispiel 3

EMIT®-Prinzip

Zu 2,00 ml 50 mMol/l K-Phosphatpuffer (pH 7,5) mit 10 U/ml Creatinin-Iminohydrolase werden hinzugemischt:

0,02 ml Probe (oder $H_2O$ für den Leerwert)
1,00 ml 0,7 mMol/l Oxalessigsäure in Pufferlösung
(50 mMol/l Phosphat, pH 7,5) 0,01 ml
4 · $10^{-7}$ Mol/l Methylhydantoin-Edestin-Antiserum
(Bindungsstellenkonzentration)
0,04 ml 1,4 · $10^{-2}$ Mol/l NADH

Nach 20 bis 30 Minuten Inkubation bei 30° C werden hinzugefügt:

0,05 ml 1 · $10^{-8}$ Mol/l 1-Methylhydantoin-Malatdehydrogenase-Konjugat

und die Enzymaktivität als $\Delta$E/min spektrophotometrisch bei 340 nm und 30° C gemessen.

## Patentansprüche

1. Verfahren zur immunologischen Bestimmung von Creatinin, dadurch gekennzeichnet, daß man Creatinin in 1-Methylhydantoin umwandelt, das gebildete 1-Methylhydantoin mit Antikörpern, die gegen ein Konjugat von einem ersten Hydantoin der allgemeinen Formel I

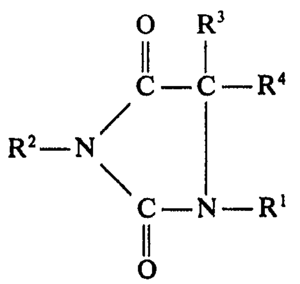

in der $R^1$, $R^2$, $R^3$ und $R^4$ unabhängig voneinander je ein H-Atom, einen Alkylrest mit 1 bis 3 C-Atomen oder einen Phenylrest bedeuten, mit einer zur Antikörperbildung geeigneten ersten Haptenträgersubstanz gerichtet sind, in wäßrigem Medium inkubiert, mit einem Konjugat von einem zweiten Hydantoin der allgemeinen Formel I mit einer zweiten Haptenträgersubstanz umsetzt, wobei eine der Komponenten Antikörper und Konjugat in fester Phase oder gelöster Form, die andere Komponente in gelöster Form vorliegt, und die Hemmung der Bindungsreaktion zwischen den Antikörpern und dem Hydantoin-Konjugat mit der zweiten Haptenträgersubstanz mißt.

2. Verfahren nach Anspruch 1, dadurch gekennzeichnet, daß die Umwandlung des Creatinins in 1-Methylhydantoin in wäßriger Lösung vor oder während der Inkubation mit den Antikörpern durchgeführt wird.

3. Verfahren nach Anspruch 1 und 2, dadurch gekennzeichnet, daß man das Creatinin enzymatisch in 1-Methylhydantoin unwandelt.

4. Verfahren nach Anspruch 3, dadurch gekennzeichnet, daß man das Creatinin mit Creatinin-Iminohydrolase (EC. 3.5.4.21) in 1-Methylhydantoin umwandelt.

5. Verfahren nach Anspruch 1, dadurch gekennzeichnet, daß das erste Hydantoin mit dem zweiten Hydantoin identisch ist.

6. Verfahren nach Anspruch 5, dadurch gekennzeichnet, daß man als erstes und zweites Hydantoin 1-Methylhydantoin ($R^1 = CH_3$, $R^2 - R^4 = -H$) verwendet.

7. Verfahren nach Anspruch 1, dadurch gekennzeichnet, daß man als Haptenträgersubstanzen Proteine, Polysaccharide, Lipopolysaccharide, Latex-Partikel, Aktivkohle, Polylysin oder Viren verwendet.

8. Verfahren nach Anspruch 7, dadurch gekennzeichnet, daß man als Protein Humanserumalbumin, Rinderserumalbumin, $\beta$-Galactosidase oder Edestin verwendet.

9. Verfahren nach einem der Ansprüche 7 und 8, dadurch gekennzeichnet, daß die erste Haptenträgersubstanz von der zweiten Haptenträgersubstanz verschieden ist.

10. Verfahren nach Anspruch 9, dadurch gekennzeichnet, daß man als erste Haptenträgersubstanz Edestin verwendet.

11. Verfahren nach Anspruch 10, dadurch gekennzeichnet, daß man als zweite Haptenträgersubstanz Humanserumalbumin, Rinderserumalbumin, $\beta$-Galactosidase oder Latex verwendet.

12. Verfahren nach Anspruch 1, dadurch gekennzeichnet, daß man über aliphatische oder araliphatische Carbonsäuren als Brückenglieder an die Haptenträgersubstanzen gebundene Hydantoine verwendet.

13. Verfahren nach Anspruch 12, dadurch gekennzeichnet, daß man Konjugate verwendet, deren Carbonsäurebrücke mindestens 2 Kohlenstoffatome enthält.

14. Verfahren nach Anspruch 13, dadurch gekennzeichnet, daß man Konjugate verwendet, die als Brückenglieder eine Alkyl-Phenyl-Carbonsäure oder Oligomere davon enthalten.

15. Verfahren nach einem der Ansprüche 1 und 12 bis 14, dadurch gekennzeichnet, daß das Brückenglied des zur Antikörperbildung verwendeten Hydantoin-Konjugats von demjenigen des zum Bindungshemmtest verwendeten Hydantoin-Konjugats verschieden ist.

16. Verfahren nach den Ansprüchen 1, 12 und 15, dadurch gekennzeichnet, daß man als Brückenglied für das zur Antikörperbildung verwendete Hydantoin-Konjugat eine aliphatische Carbonsäure verwendet.

17. Verfahren nach den Ansprüchen 1, 12 und 15, dadurch gekennzeichnet, daß man als Brückenglied für das im Bindungshemmtest eingesetzte Hydantoin-Konjugat eine Alkyl-Phenyl-Carbonsäure verwendet.

18. Verfahren nach Anspruch 1, dadurch gekennzeichnet, daß man Konjugate verwendet, die pro Molekül Haptenträgersubstanz 1 bis 50 Moleküle Hydantoin enthalten.

19. Verfahren nach Anspruch 1, dadurch gekennzeichnet, daß man die Antikörper in Form eines Antiserums, einer daraus gewonnenen Immunglobulinfraktion, als monoclonale Antikörper oder als Antikörperfragmente einsetzt.

20. Verfahren nach Anspruch 1, dadurch gekennzeichnet, daß man die Hemmung der Bindungsreaktion durch nephelometrische oder turbimetrische Messung der Immunpräzipitation in einem vorgesehenen Zeitintervall bestimmt.

21. Verfahren nach Anspruch 1, dadurch gekennzeichnet, daß man die Hemmung der Bindungsreaktion durch Rücktitration von nichtgebundenem Haptenträgersubstanz-Hydantoin-Konjugat mit markierten Antikörpern und Messung des gebundenen oder des nichtgebundenen Anteils der markierten Substanz bestimmt.

22. Verfahren nach Anspruch 21, dadurch gekennzeichnet, daß man enzym- oder coenzymmarkierte Antikörper verwendet.

23. Verfahren nach Anspruch 21, dadurch gekennzeichnet, daß man radioaktiv- oder fluoreszenzmarkierte Antikörper verwendet.

24. Verfahren nach einem der vorhergehenden Ansprüche, dadurch gekennzeichnet, daß man Schafantikörper verwendet.

25. Verfahren nach Anspruch 1, dadurch gekennzeichnet, daß man zur Gewinnung der Antikörper das entsprechende Konjugat zusammen mit dem Freundschen Adjuvans appliziert.

26. Verfahren nach Anspruch 1 oder 25, dadurch gekennzeichnet, daß man zur Antikörpergewinnung ein Hydantoin-Konjugat verwendet, welches eine Haptenträgersubstanz enthält, die mit der zur Antikörpergewinnung verwendeten Haptenträgersubstanz kreuzreagiert, freie Haptenträgersubstanz mit der Antikörperlösung mischt, den dabei gebildeten Niederschlag abtrennt und den erhaltenen Überstand als Antikörperlösung im Test einsetzt.

27. Reagenz zur immunologischen Bestimmung von Creatinin, dadurch gekennzeichnet, daß es Creatinin-Iminohydrolase, Antikörper gegen ein Konjugat eines Hydantoins der allgemeinen Formel I

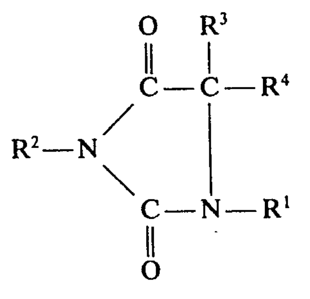

in der $R^1$, $R^2$, $R^3$ und $R^4$ unabhängig voneinander je ein H-Atom, einen Alkylrest mit 1 bis 3 C-Atomen oder einen Phenylrest bedeuten, mit einer ersten Haptenträgersubstanz, ein Konjugat eines Hydantoins der allgemeinen Formel I mit einer zweiten Haptenträgersubstanz, die mit der ersten Haptenträgersubstanz nicht wesentlich kreuzreagiert, und Puffersubstanz enthält.

28. Reagenz nach Anspruch 27, dadurch gekennzeichnet, daß es Antikörper gegen 1-Methylhydantoin und ein Konjugat von 1-Methylhydantoin mit einer Haptenträgersubstanz enthält.

29. Reagenz nach Anspruch 27 oder 28, dadurch gekennzeichnet, daß es Polyäthylenglykol enthält.

30. Reagenz nach einem der Ansprüche 27 bis 29, dadurch gekennzeichnet, daß die Antikörper als Antiserum, Immunglobulinfraktion, Antikörperfragmente oder monoklonale Antikörper vorliegen.

Beispiel 3

EMIT®-Prinzip

Zu 2,00 ml 50 mMol/l K-Phosphatpuffer (pH 7,5) mit 10 U/ml Creatinin-Iminohydrolase werden hinzugemischt:

0,02 ml Probe (oder $H_2O$ für den Leerwert)
1,00 ml 0,7 mMol/l Oxalessigsäure in Pufferlösung
(50 mMol/l Phosphat, pH 7,5) 0,01 ml
$4 \cdot 10^{-7}$ Mol/l Methylhydantoin-Edestin-Antiserum
(Bindungsstellenkonzentration)
0,04 ml $1,4 \cdot 10^{-2}$ Mol/l NADH

Nach 20 bis 30 Minuten Inkubation bei 30° C werden hinzugefügt:

0,05 ml $1 \cdot 10^{-8}$ Mol/l 1-Methylhydantoin-Malatdehydrogenase-Konjugat

und die Enzymaktivität als $\Delta$E/min spektrophotometrisch bei 340 nm und 30° C gemessen.

**Patentansprüche**

1. Verfahren zur immunologischen Bestimmung von Creatinin, dadurch gekennzeichnet, daß man Creatinin in 1-Methylhydantoin umwandelt, das gebildete 1-Methylhydantoin mit Antikörpern, die gegen ein Konjugat von einem ersten Hydantoin der allgemeinen Formel I

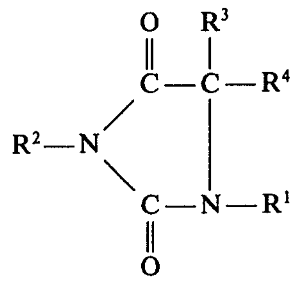

in der $R^1$, $R^2$, $R^3$ und $R^4$ unabhängig voneinander je ein H-Atom, einen Alkylrest mit 1 bis 3 C-Atomen oder einen Phenylrest bedeuten, mit einer zur Antikörperbildung geeigneten ersten Haptenträgersubstanz gerichtet sind, in wäßrigem Medium inkubiert, mit einem Konjugat von einem zweiten Hydantoin der allgemeinen Formel I mit einer zweiten Haptenträgersubstanz umsetzt, wobei eine der Komponenten Antikörper und Konjugat in fester Phase oder gelöster Form, die andere Komponente in gelöster Form vorliegt, und die Hemmung der Bindungsreaktion zwischen den Antikörpern und dem Hydantoin-Konjugat mit der zweiten Haptenträgersubstanz mißt.

2. Verfahren nach Anspruch 1, dadurch gekennzeichnet, daß die Umwandlung des Creatinins in 1-Methylhydantoin in wäßriger Lösung vor oder während der Inkubation mit den Antikörpern durchgeführt wird.

3. Verfahren nach Anspruch 1 und 2, dadurch gekennzeichnet, daß man das Creatinin enzymatisch in 1-Methylhydantoin unwandelt.

4. Verfahren nach Anspruch 3, dadurch gekennzeichnet, daß man das Creatinin mit Creatinin-Iminohydrolase (EC. 3.5.4.21) in 1-Methylhydantoin umwandelt.

5. Verfahren nach Anspruch 1, dadurch gekennzeichnet, daß das erste Hydantoin mit dem zweiten Hydantoin identisch ist.

6. Verfahren nach Anspruch 5, dadurch gekennzeichnet, daß man als erstes und zweites Hydantoin 1-Methylhydantoin ($R^1 = CH_3$, $R^2 - R^4 = -H$) verwendet.

7. Verfahren nach Anspruch 1, dadurch gekennzeichnet, daß man als Haptenträgersubstanzen Proteine, Polysaccharide, Lipopolysaccharide, Latex-Partikel, Aktivkohle, Polylysin oder Viren verwendet.

8. Verfahren nach Anspruch 7, dadurch gekennzeichnet, daß man als Protein Humanserumalbumin, Rinderserumalbumin, $\beta$-Galactosidase oder Edestin verwendet.

9. Verfahren nach einem der Ansprüche 7 und 8, dadurch gekennzeichnet, daß die erste Haptenträgersubstanz von der zweiten Haptenträgersubstanz verschieden ist.

10. Verfahren nach Anspruch 9, dadurch gekennzeichnet, daß man als erste Haptenträgersubstanz Edestin verwendet.

11. Verfahren nach Anspruch 10, dadurch gekennzeichnet, daß man als zweite Haptenträgersubstanz Humanserumalbumin, Rinderserumalbumin, $\beta$-Galactosidase oder Latex verwendet.

12. Verfahren nach Anspruch 1, dadurch gekennzeichnet, daß man über aliphatische oder araliphatische Carbonsäuren als Brückenglieder an die Haptenträgersubstanzen gebundene Hydantoine verwendet.

13. Verfahren nach Anspruch 12, dadurch gekennzeichnet, daß man Konjugate verwendet, deren Carbonsäurebrücke mindestens 2 Kohlenstoffatome enthält.

14. Verfahren nach Anspruch 13, dadurch gekennzeichnet, daß man Konjugate verwendet, die als Brückenglieder eine Alkyl-Phenyl-Carbonsäure oder Oligomere davon enthalten.

15. Verfahren nach einem der Ansprüche 1 und 12 bis 14, dadurch gekennzeichnet, daß das Brückenglied des zur Antikörperbildung verwendeten Hydantoin-Konjugats von demjenigen des zum Bindungshemmtest verwendeten Hydantoin-Konjugats verschieden ist.

16. Verfahren nach den Ansprüchen 1, 12 und 15, dadurch gekennzeichnet, daß man als Brückenglied für das zur Antikörperbildung verwendete Hydantoin-Konjugat eine aliphatische Carbonsäure verwendet.

17. Verfahren nach den Ansprüchen 1, 12 und 15, dadurch gekennzeichnet, daß man als Brückenglied für das im Bindungshemmtest eingesetzte Hydantoin-Konjugat eine Alkyl-Phenyl-Carbonsäure verwendet.

18. Verfahren nach Anspruch 1, dadurch gekennzeichnet, daß man Konjugate verwendet, die pro Molekül Haptenträgersubstanz 1 bis 50 Moleküle Hydantoin enthalten.

19. Verfahren nach Anspruch 1, dadurch gekennzeichnet, daß man die Antikörper in Form eines Antiserums, einer daraus gewonnenen Immunglobulinfraktion, als monoclonale Antikörper oder als Antikörperfragmente einsetzt.

20. Verfahren nach Anspruch 1, dadurch gekennzeichnet, daß man die Hemmung der Bindungsreaktion durch nephelometrische oder turbimetrische Messung der Immunpräzipitation in einem vorgesehenen Zeitintervall bestimmt.

21. Verfahren nach Anspruch 1, dadurch gekennzeichnet, daß man die Hemmung der Bindungsreaktion durch Rücktitration von nichtgebundenem Haptenträgersubstanz-Hydantoin-Konjugat mit markierten Antikörpern und Messung des gebundenen oder des nichtgebundenen Anteils der markierten Substanz bestimmt.

22. Verfahren nach Anspruch 21, dadurch gekennzeichnet, daß man enzym- oder coenzymmarkierte Antikörper verwendet.

23. Verfahren nach Anspruch 21, dadurch gekennzeichnet, daß man radioaktiv- oder fluoreszenzmarkierte Antikörper verwendet.

24. Verfahren nach einem der vorhergehenden Ansprüche, dadurch gekennzeichnet, daß man Schafantikörper verwendet.

25. Verfahren nach Anspruch 1, dadurch gekennzeichnet, daß man zur Gewinnung der Antikörper das entsprechende Konjugat zusammen mit dem Freundschen Adjuvans appliziert.

26. Verfahren nach Anspruch 1 oder 25, dadurch gekennzeichnet, daß man zur Antikörpergewinnung ein Hydantoin-Konjugat verwendet, welches eine Haptenträgersubstanz enthält, die mit der zur Antikörpergewinnung verwendeten Haptenträgersubstanz kreuzreagiert, freie Haptenträgersubstanz mit der Antikörperlösung mischt, den dabei gebildeten Niederschlag abtrennt und den erhaltenen Überstand als Antikörperlösung im Test einsetzt.

27. Reagenz zur immunologischen Bestimmung von Creatinin, dadurch gekennzeichnet, daß es Creatinin-Iminohydrolase, Antikörper gegen ein Konjugat eines Hydantoins der allgemeinen Formel I

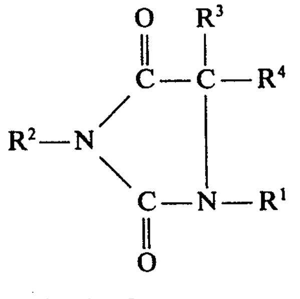

in der $R^1$, $R^2$, $R^3$ und $R^4$ unabhängig voneinander je ein H-Atom, einen Alkylrest mit 1 bis 3 C-Atomen oder einen Phenylrest bedeuten, mit einer ersten Haptenträgersubstanz, ein Konjugat eines Hydantoins der allgemeinen Formel I mit einer zweiten Haptenträgersubstanz, die mit der ersten Haptenträgersubstanz nicht wesentlich kreuzreagiert, und Puffersubstanz enthält.

28. Reagenz nach Anspruch 27, dadurch gekennzeichnet, daß es Antikörper gegen 1-Methylhydantoin und ein Konjugat von 1-Methylhydantoin mit einer Haptenträgersubstanz enthält.

29. Reagenz nach Anspruch 27 oder 28, dadurch gekennzeichnet, daß es Polyäthylenglykol enthält.

30. Reagenz nach einem der Ansprüche 27 bis 29, dadurch gekennzeichnet, daß die Antikörper als Antiserum, Immunglobulinfraktion, Antikörperfragmente oder monoklonale Antikörper vorliegen.

31. Reagenz nach Anspruch 30, dadurch gekennzeichnet, daß die Antikörper markiert sind.

32. Reagenz nach den Ansprüchen 28 bis 31, dadurch gekennzeichnet, daß es 0,05 bis 100 U/ml Creatininiminohydrolase, 0,5 bis 500 µg/ml Konjugat aus 1-Methylhydantoin und Serumalbumin im molaren Verhältnis 1-Methylhydantoin : Serumalbumin von 2 : 1 bis 30 : 1, Antikörper : 1-Methylhydantoin-Konjugat im Molverhältnis 0,1 bis 10, bezogen auf die Haptenbindungsstellen-Konzentration, 0,1 bis 8 Gew.-% Polyäthylenglykol und Puffersubstanz der Ionenstärke 0,03 bis 0,4, pH 4 bis 10, enthält.

33. Reagenz nach den Ansprüchen 28 bis 31, dadurch gekennzeichnet, daß es 0,05 bis 100 U/ml Creatinin-Iminohydrolase, $10^{-4}$ bis $10^{14}$ Mol/l Antikörper gegen 1-Methylhydantoin-Haptenträgersubstanz-Konjugat, bezogen auf aktive Hapten-Rezeptorstellen, $10^{-4}$ bis $10^{-14}$ Mol/l 1-Methylhydantoin-Malatdehydrogenase-Konjugat, 0,05 bis 50 mMol/l Oxalessigsäure, 5 bis 200 mMol/l Phosphatpuffer pH 6 bis 8,5 und 0,05 bis 0,4 mMol/l NADH enthält.

**Claims**

1. Process for the immunological determination of creatinine, characterised in that one converts creatinine into 1-methylhydantoin, incubates the 1-methylhydantoin formed in an aqueous medium with antibodies which are directed against a conjugate of a first hydantoin of the general formula I

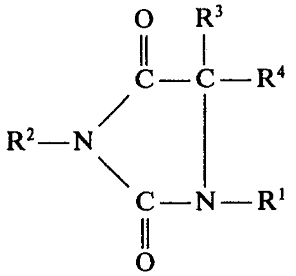

in which $R^1$, $R^2$, $R^3$ and $R^4$, independently of each other, each signify an H-atom, an alkyl radical with 1 to 3 C-atoms or a phenyl radical, with a first hapten carrier substance suitable for the antibody formation, reacts with a conjugate of a second hydantoin of the general formula I with a second hapten carrier substance, whereby one of the components antibody and conjugate is present in solid phase or in dissolved form, the other component is present in dissolved form, and measures the inhibition of the binding reaction between the antibodies and the hydantoin conjugate with the second hapten carrier substance.

2. Process according to claim 1, characterised in that the conversion of the creatinine into 1-methylhydantoin is carried out in aqueous solution before or during the incubation with the antibodies.

3. Process according to claim 1 and 2, characterised in that one converts the creatinine enzymatically into 1-methylhydantoin.

4. Process according to claim 3, characterised in that one converts the creatinine into 1-methylhydantoin with creatinine iminohydrolase (EC 3.5.4.21).

5. Process according to claim 1, characterised in that the first hydantoin is identical with the second hydantoin.

6. Process according to claim 5, characterised in that as first and second hydantoin one uses 1-methylhydantoin ($R^1 = CH_3$; $R^2 - R^4 = H$).

7. Process according to claim 1, characterised in that as hapten carrier substances one uses proteins, polysaccharides, lipopolysaccharides, latex particles, active charcoal, polylysine or viruses.

8. Process according to claim 7, characterised in that as protein one uses human serum albumin, bovine serum albumin, $\beta$-galactosidase or edestin.

9. Process according to one of claims 7 and 8, characterised in that the first hapten carrier substance is different from the second hapten carrier substance.

10. Process according to claim 9, characterised in that one uses edestin as first hapten carrier substance.

11. Process according to claim 10, characterised in that as second hapten carrier substance one uses human serum albumin, bovine serum albumin, $\beta$-galactosidase or latex.

12. Process according to claim 1, characterised in that one uses hydantoins connected to the hapten carrier substances via aliphatic or araliphatic carboxylic acids as bridge members.

13. Process according to claim 12, characterised in that one uses conjugates, the carboxylic acid bridge of which contains at least 2 carbon atoms.

14. Process according to claim 13, characterised in that one uses conjugates which , as bridge member, contain an alkylphenylcarboxylic acid or on oligomer thereof.

15. Process according to one of claims 1 and 12 to 14, characterised in that the bridge member of the hydantoin conjugate used for the antibody formation is different from that of the hydantoin conjugate used for the binding inhibition test.

16. Process according to claim 1, 12 and 15, characterised in that, as bridge member for the hydantoin conjugate used for the antibody formation, one uses an aliphatic carboxylic acid.

17. Process according to claims 1, 12 and 15, characterised in that, as bridge member for the hydantoin conjugate used in the binding inhibition test, one uses an alkylphenylcarboxylic acid.

18. Process according to claim 1, characterised in that one uses conjugates which, per molecule of hapten carrier substance, contain 1 to 50 molecules of hydantoin.

19. Process according to claim 1, characterised in that one uses the antibodies in the form of an antiserum, of an immunoglobulin fraction obtained therefrom, as monoclonal antibodies or as antibody fragments.

20. Process according to claim 1, characterised in that one determines the inhibition of the binding reaction by nephelometric or turbidimetric measurement of the immunoprecipitation in a predetermined time interval.

21. Process according to claim 1, characterised in that one determines the inhibition of the binding reaction by backtitration of non-bound hapten carrier substance-hydantoin conjugate with marked antibodies and measurement of the bound or non-bound portion of the marked substance.

22. Process according to claim 21, characterised in that one uses enzyme- or coenzyme-marked antibodies.

23. Process according to claim 21, characterised in that one uses radio-active- or fluorescent-marked antibodies.

24. Process according to any of the preceding claims, characterised in that one uses sheep antibodies.

25. Process according to claim 1, characterised in that, for the obtaining of the antibodies, one administers the corresponding conjugate, together with Freund's adjuvant.

26. Process according to claim 1 or 25, characterised in that for obtaining the antibodies one uses a hydantoin conjugate which contains a hapten carrier substance which cross-reacts with the hapten carrier substance used for obtaining the antibodies, mixes free hapten carrier substance with the antibody solution, separates off the precipitate thereby formed and uses the supernatant obtained in the test as antibody solution.

27. Reagent for the immunological determination of creatinine, characterised in that it contains creatinine iminohydrolase, antibodies against a conjugate of a hydantoin of the general formula I

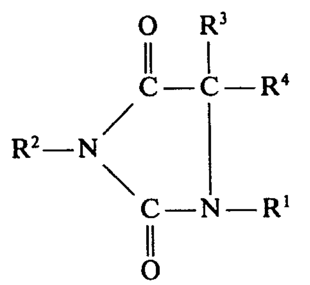

in which $R^1$, $R^2$, $R^3$ and $R^4$ independently of each other, each signify an H-atom, an alkyl radical with 1 to 3 C-atoms or a phenyl radical, with a first hapten carrier substance, a conjugate of a hydantoin of the general formula I with a second hapten carrier substance, which does not cross-react substantially with the first hapten carrier substance, and buffer substance.

28. Reagent according to claim 27, characterised in that it contains antibodies against 1-methylhydantoin and a conjugate of 1-methylhydantoin with a hapten carrier substance.

29. Reagent according to claim 27 or 28, characterised in that it contains polyethylene glycol.

30. Reagent according to one of claims 27 to 29, characterised in that the antibodies are present as antiserum, immunoglobulin fraction, antibody fragments or monoclonal antibodies.

31. Reagent according to claim 30, characterised in that the antibodies are marked.

32. Reagent according to claims 28 to 31, characterised in that it contains 0.05 to 100 U/ml. creatinine iminohydrolase, 0.5 to 500 μg./ml. conjugate of 1-methylhydantoin and serum albumin in the molar ratio of 1-methylhydantoin : serum albumin of 2 : 1 to 30 : 1, antibody : 1-methylhydantoin conjugate in the mole ratio of 0.1 to 10, referred to the hapten binding point concentration, 0.1 to 8 wt.% of polyethylene glycol and buffer substance of the ionic strength of 0.03 to 0.4, pH 4 to 10.

33. Reagent according to claims 28 to 31, characterised in that it contains 0.05 to 100 U/ml. creatinine iminohydrolase, $10^{-4}$ to $10^{-14}$ mole/l. of antibodies against 1-methylhydantoin-hapten carrier substance conjugate, referred to the active hapten receptor points, $10^{-4}$ to $10^{-14}$ mole/l. 1-methylhydantoin-malate dehydrogenase conjugate, 0.05 to 50 mMole/l. oxalacetic acid, 5 to 200 mMole/l. of phosphate buffer pH 6 to 8.5 and 0.05 to 0.4 mMole/l. NADH.

**Revendications**

1. Procédé pour la détermination immunologique de la créatinine, caractérise en ce que l'on transforme la créatinine en 1-méthylhydantoïne, on incube en milieu aqueux la 1-méthylhydantoïne formée avec des anticorps qui sont dirigés contre un conjugué d'une première hydantoïne de formule générale I

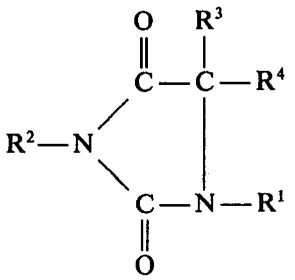

dans laquelle $R^1$, $R^2$, $R^3$ et $R^4$, indépendamment l'un de l'autre, représentent chacun un atome de H, un radical alcoyle avec 1 à 3 atomes de C ou un radical phényle, avec une première substance support d'haptène convenant à la formation d'anticorps, fait réagir avec un conjugué d'une deuxième hydantoïne de formule générale I avec une deuxième substance support d'haptène, l'un des composants anticorps et conjugué se présentant sous forme solide ou dissoute, l'autre composant sous forme dissoute, et on mesure l'inhibition de la réaction de liaison entre les anticorps et le conjugué d'hydantoïne avec la deuxième substance support d'haptène.

2. Procédé selon la revendication 1, caractérisé en ce que la transformation de la créatinine en 1-méthylhydantoïne est effectuée en solution aqueuse avant ou pendant l'incubation avec les anticorps.

3. Procédé selon la revendication 1 et 2, caractérisé en ce que l'on transforme la créatinine en 1-méthylhydantoïne enzymatiquement.

4. Procédé selon la revendication 3, caractérisé en ce qu'on transforme la créatinine en 1-méthylhydantoïne au moyen de créatinine-iminohydolase (EC. 3.5.4.21).

5. Procédé selon la revendication 1, caractérisé en ce que la première hydantoïne est identique à la deuxième hydantoïne.

6. Procédé selon la revendication 5, caractérisé en ce que l'on utilise en tant que première et deuxième hydantoïne la 1-méthylhydantoïne ($R^1 = CH_3$, $R^2 - R^4 = -H$).

7. Procédé selon la revendication 1, caractérisé en ce que l'on utilise en tant que substances support d'haptène des protéines, des polysaccharides, des lipopolysaccharides, des particules de latex, du charbon actif, de la polylysine ou des virus.

8. Procédé selon la revendication 7, caractérisé en ce que l'on utilise en tant que protéine de la sérumalbumine humaine, de la sérumalbumine de bovins, de la $\beta$-galactosidase ou de l'édestine.

9. Procédé selon l'une des revendications 7 et 8, caractérisé en ce que la première substance support d'haptène est différente de la deuxième substance support d'haptène.

10. Procédé selon la revendication 9, caractérisé en ce que l'on utilise en tant que première substance support d'haptène, de l'édestine.

11. Procédé selon la revendication 10, caractérisé en ce que l'on utilise en tant que deuxième substance support d'haptène, de la sérumalbumine humaine, de la sérumalbumine de bovins, de la $\beta$-galactosidase ou du latex.

12. Procédé selon la revendication 1, caractérisé en ce que l'on utilise des hydantoïnes liées aux substances support d'haptène par l'intermédiaire d'acides carboxyliques aliphatiques ou araliphatiques en tant qu'éléments de pontage.

13. Procédé selon la revendication 12, caractérisé en ce que l'on utilise un conjugué dont le pont acide carboxylique contient au moins 2 atomes de carbone.

14. Procédé selon la revendication 13, caractérisé en ce que l'on utilise des conjugués qui contiennent en tant qu'éléments de pontage, un acide alcoylphényl-carboxylique ou des oligomères de celui-ci.

15. Procédé selon l'une des revendications 1 et 12 à 14, caractérisé en ce que l'élément de pontage du conjugué d'hydantoïne utilisé pour la formation d'anticorps est différent de celui du conjugué d'hydantoïne utilisé pour l'essai d'inhibition de liaison.

16. Procédé selon l'une des revendications 1, 12 et 15, caractérisé en ce que l'on utilise, en tant qu'élélement de pontage pour le conjugué d'hydantoïne utilisé pour la formation d'anticorps, un acide carboxylique aliphatique.

17. Procédé selon l'une des revendications 1, 12 et 15, caractérisé en ce que l'on utilise en tant qu'élément de pontage pour le conjugué d'hydantoïne mis en oeuvre dans l'essai d'inhibition de liaison, un acide alcoyl-phényl-carboxylique.

18. Procédé selon la revendication 1, caractérisé en ce que l'on utilise des conjugués qui contiennent par molécule de substance support d'haptène 1 à 50 molécules d'hydantoïne.

19. Procédé selon la revendication 1, caractérisé en ce que l'on met en oeuvre les anticorps sous forme d'un antisérum, d'une fraction d'immunoglobuline obtenue à partir de celui-ci, sous forme d'anticorps monoclonaux ou sous forme de fragments d'anticorps.

20. Procédé selon la revendication 1, caractérisé en ce que l'on détermine l'inhibition de la réaction de liaison par mesure néphélométrique ou turbimétrique de l'immunoprécipitation dans un intervalle de temps préétabli.

21. Procédé selon la revendication 1, caractérisé en ce que l'on détermine l'inhibition de la réaction de liaison par titrage en retour du conjugué substance support d'haptène-hydantoïne non lié avec des anticorps marqués et mesure de la proportion liée ou de la proportion non liée de la substance marquée.

22. Procédé selon la revendication 21, caractérisé en ce que l'on utilise des anticorps marqués par des enzymes ou par des co-enzymes.

23. Procédé selon la revendication 21, caractérisé en ce que l'on utilise des anticorps marqués radioactivement ou par fluorescence.

24. Procédé selon l'une des revendications précédentes, caractérisé en ce que l'on utilise des anticorps de mouton.

25. Procédé selon la revendication 1, caractérisé en ce que, pour obtenir les anticorps, on applique le conjugué correspondant ensemble avec l'adjuvant de Freund.

26. Procédé selon la revendication 1 ou 25, caractérisé en ce que l'on utilise pour l'obtention d'anticorps, un conjugué d'hydantoïne qui contient une substance support d'haptène qui réagit de façon croisée avec la substance support d'haptène utilisée pour l'obtention d'anticorps, mélange la substance support d'haptène libre avec la solution d'anticorps, sépare le précipité alors formé et met en oeuvre dans l'essai le surnageant obtenu en tant que solution d'anticorps.

27. Réactif pour la détermination immunologique de la créatinine, caractérisé en ce qu'il contient de la créatinine-iminohydrolase, des anticorps contre un conjugué d'une hydantoïne de formule générale I

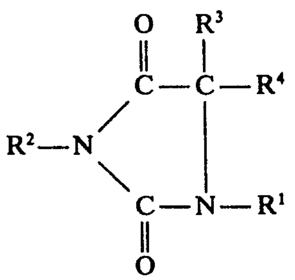

dans laquelle $R^1$, $R^2$, $R^3$ et $R^4$, indépendamment l'un de l'autre, représentent chacun un atome de H, un radical alcoyle avec 1 à 3 atomes de C ou un radical phényle, avec une première substance support d'haptène, un conjugué d'une hydantoïne de formule générale I avec une deuxième substance support d'haptène qui ne réagit pas notablement de façon croisée avec la première substance support d'haptène, et une substance tampon.

28. Réactif selon la revendication 27, caractérisé en ce qu'il contient des anticorps contre la 1-méthylhydantoïne et un conjugué de la 1-méthylhydantoïne avec une substance support d'haptène.

29. Réactif selon la revendication 27 ou 28, caractérisé en ce qu'il contient du polyéthylèneglycol.

30. Réactif selon l'une des revendications 27 à 29, caractérisé en ce que les anticorps se présentent sous forme d'antisérum, de fraction d'immunoglobuline, de fragments d'anticorps ou d'anticorps monoclonaux.

31. Réactif selon la revendication 30, caractérisé en ce que les anticorps sont marqués.

32. Réactif selon les revendications 28 à 31, caractérisé en ce qu'il contient 0,05 à 100 U/ml de créatinine-iminohydrolase, 0,5 à 500 μg/ml de conjugué de 1-méthylhydantoïne et de sérumalbumine dans le rapport molaire 1-méthylhydantoïne : sérumalbumine de 2 : 1 à 30 : 1, des anticorps du conjugué de 1-méthylhydantoïne dans le rapport molaire 0,1 à 10 par rapport à la concentration des sites de liaison de l'haptène, 0,1 à 8% en poids de polyéthylèneglycol et de la substance tampon de force ionique 0,03 à 0,4, pH 4 à 10.

33. Réactif selon les revendications 28 à 31, caractérisé en ce qu'il contient 0,05 à 100 U/ml de créatinine-iminohydrolase, $10^{-4}$ à $10^{-14}$ mole/l d'anticorps contre le conjugué 1-méthylhydantoïne-substance support d'haptène, par rapport aux sites récepteurs de l'haptène actifs, $10^{-4}$ à $10^{-14}$ mole/l de conjugué 1-méthylhydantoïne-malate-deshydrogénase, 0,05 à 50 mmoles/l d'acide oxalacétique, 5 à 200 mmoles/l de tampon phosphate, pH 6 à 8,5 et 0,05 à 0,4 mmole/l de NADH.

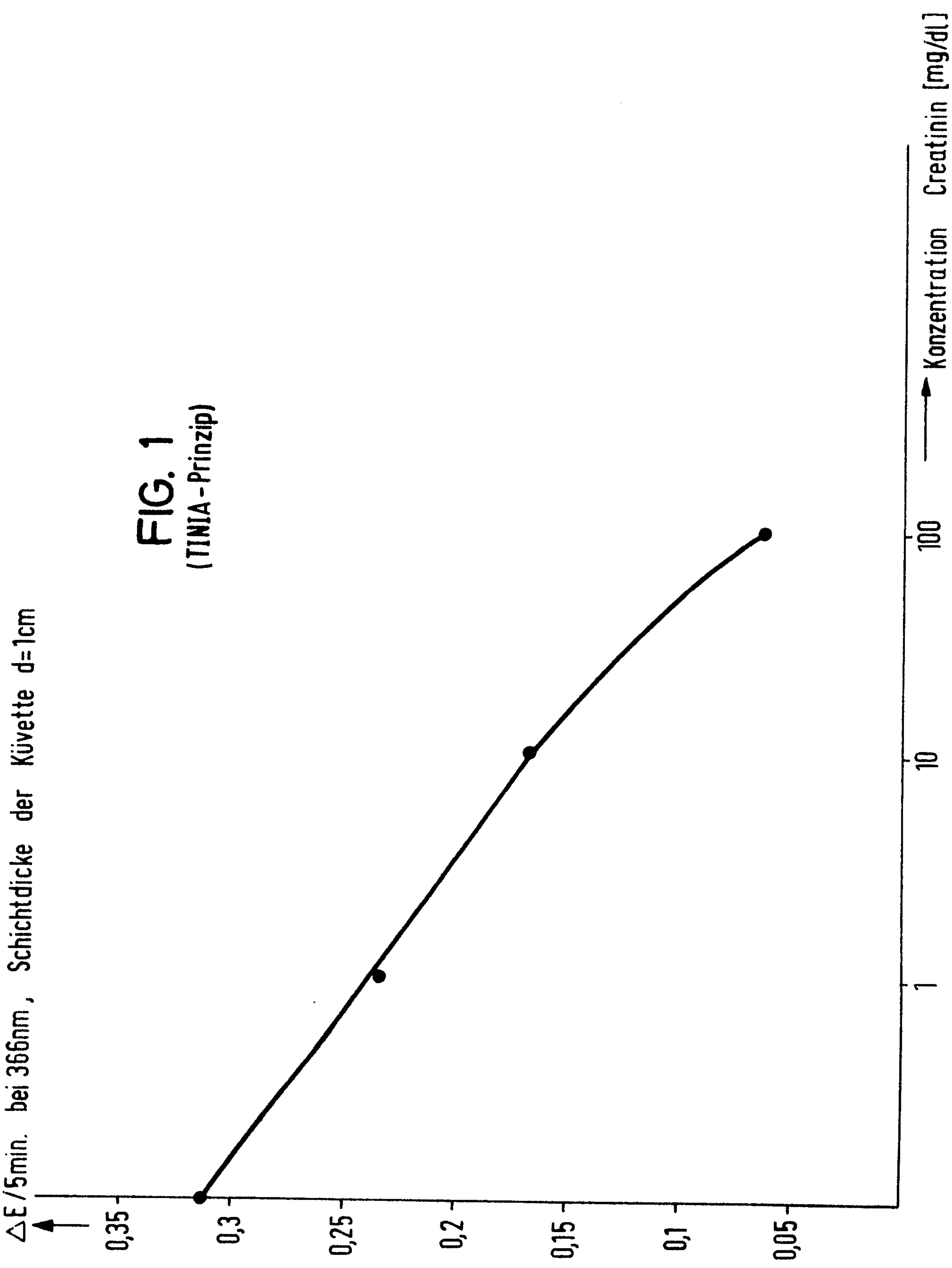


FIG. 1
(TINIA-Prinzip)

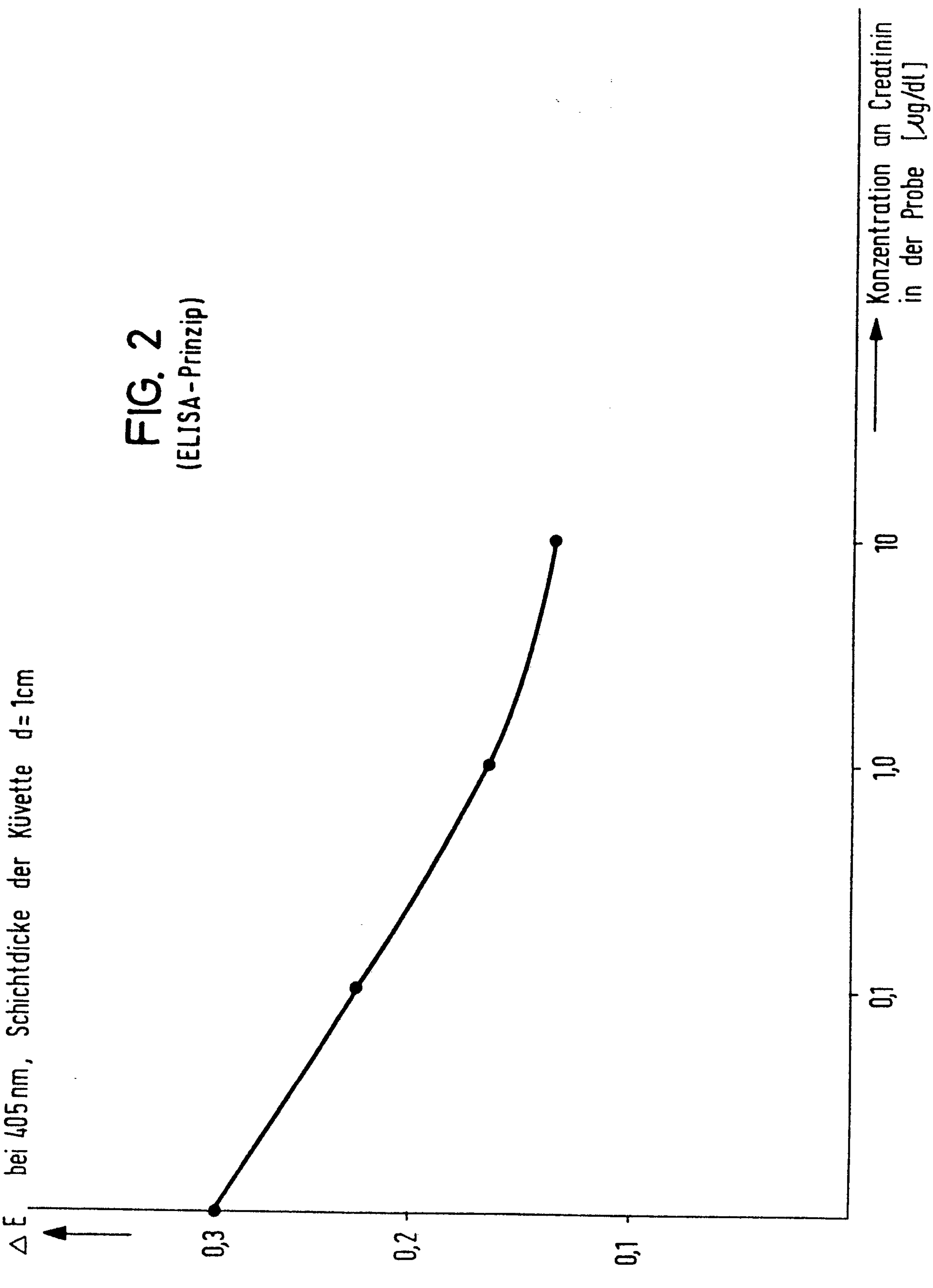


FIG. 2
(ELISA-Prinzip)